(19) Europäisches Patentamt European Patent Office Office européen des brevets

(11) **EP 2 550 919 A1**

(12) **EUROPEAN PATENT APPLICATION**

(43) Date of publication:
**30.01.2013 Bulletin 2013/05**

(51) Int Cl.:
*A61B 8/06* *(2006.01)*     *A61B 8/08* *(2006.01)*

(21) Application number: **12177809.6**

(22) Date of filing: **25.07.2012**

| | |
|---|---|
| (84) Designated Contracting States:<br>**AL AT BE BG CH CY CZ DE DK EE ES FI FR GB<br>GR HR HU IE IS IT LI LT LU LV MC MK MT NL NO<br>PL PT RO RS SE SI SK SM TR**<br>Designated Extension States:<br>**BA ME** | (72) Inventor: **Kim, Dae Young**<br>**135-851 Seoul (KR)**<br><br>(74) Representative: **Schmid, Wolfgang**<br>**Lorenz & Kollegen**<br>**Patentanwälte Partnerschaftsgesellschaft**<br>**Alte Ulmer Strasse 2**<br>**89522 Heidenheim (DE)** |
| (30) Priority: **26.07.2011 KR 20110074257** | |
| (71) Applicant: **Samsung Medison Co., Ltd.**<br>**Kangwon-do 250-875 (KR)** | |

(54) **Ultrasound system and method for correcting doppler angle**

(57) There are provided embodiments for measuring an inclination angle of an ultrasound probe to correct a Doppler angle in real time. In one embodiment, by way of non-limiting example, an ultrasound system comprises: an inclination measuring unit configured to measure an inclination angle of an ultrasound probe at a predetermined cycle to form measuring information including the inclination angle, wherein the inclination measuring unit is mounted inside or outside of the ultrasound probe; and a processing unit configured to calculate a Doppler angle correction value corresponding to the inclination angle based on the measuring information and calculate a corrected Doppler angle based on the Doppler angle correction value.

FIG. 2

<u>120</u>

**Description**

CROSS-REFERENCE TO RELATED APPLICATIONS

The present application claims priority from Korean Patent Application No. 10-2011-0074257 filed on July 26, 2011.

TECHNICAL FIELD

The present invention generally relates to ultrasound systems, and more particularly to an ultrasound system and a method for measuring an inclination angle of an ultrasound probe to correct a Doppler angle in real time.

BACKGROUND

An ultrasound system is widely used in the medical applications for acquiring information of inner parts of living bodies due to its non-invasive and non-destructive nature. The ultrasound system can provide high dimensional real-time ultrasound images of the inner parts of the living bodies without any surgical operation. Thus, the ultrasound system is very important in medical applications.

The ultrasound system transmits ultrasound signals to the living bodies including target objects (e.g., blood flows, hearts, etc.) via an ultrasound probe. It then receives ultrasound echo signals reflected from the living bodies to thereby provide ultrasound images. Especially, the ultrasound system provides Doppler mode images by using the Doppler effect. In the color Doppler mode images, velocities of the target objects are represented by Doppler spectrums or colors. The Doppler mode images include Doppler spectrum images or color Doppler images.

The ultrasound probe transmits the ultrasound signals and receives the echo signals. A front end of the ultrasound system converts the received echo signals into digital signals. Receive-focused signals are formed by receive (Rx) focusing the digital signals. The receive-focused signals are bandwidth-converted by using a mixer and converted into in-phase/quadrature (IQ) signals by using appropriate decimation. The IQ signals are referred to as baseband IQ signals.

The IQ signals are represented as equation 1 provided below.

$$X_{IQ} = C + F + N \qquad (1)$$

wherein "$X_{IQ}$" denotes the IQ signal, "C" denotes the clutter signal produced by tissues of the target objects, "F" denotes the flow signal produced by the blood flow, and "N" denotes the noise produced by the ultrasound system and outside of the ultrasound system.

A user acquires velocities of the blood flow by using the ultrasound system. The Doppler formula is used to calculate the velocities of the blood flow. The Doppler formula is represented as equation 2 provided below.

$$f_D = \frac{2f_0 v \cos\theta}{c} \qquad (2)$$

wherein "$f_D$" denotes the Doppler frequency, "$f_0$" denotes an ultrasound frequency transmitted from the ultrasound probe, "v" denotes the velocity of the blood flow, "C" denotes the velocities of sound, and "$\theta$" denotes an angle between a moving path of the blood flow and a beam direction transmitted from the ultrasound probe. The "$\theta$" is referred to as the Doppler angle.

Equation 2 is reformulated in terms of "v" (e.g., the velocity of the blood flow) as equation 3 provided below.

$$v = \frac{cf_D}{2f_0 \cos\theta} \qquad (3)$$

In equation 3, the Doppler angle "$\theta$" is an important variable for determining the velocities of the blood flow.

Conventionally, the user sets the Doppler angle by using a sample-volume angle setting function in the ultrasound system. That is, the user inputs the angle between the moving path of the blood flow and the beam direction transmitted from the ultrasound probe. As a result, there is a disadvantage since it is required to frequently set the sample-volume angle during observation to adjust a difference of the angle between the moving path of the blood flow and the beam direction transmitted from the ultrasound probe, wherein the difference of the angle is caused by tilting of the ultrasound probe.

SUMMARY

There is provided an embodiment for measuring an inclination angle of an ultrasound probe by using an inclination measuring unit mounted inside or outside of the ultrasound probe and correcting a Doppler angle based on the inclination angle in real time.

In one embodiment, by way of non-limiting example, an ultrasound system may include: an inclination measuring unit configured to measure an inclination angle of an ultrasound probe at a predetermined cycle to form measuring information including the inclination angle, wherein the inclination measuring unit is mounted inside or outside of the ultrasound probe; and a processing unit configured to calculate a Doppler angle correction value corresponding to the inclination angle based on the measuring information and calculate a corrected Doppler angle based on the Doppler angle correction value.

In another embodiment, a method of correcting a Doppler angle may comprise: measuring an inclination angle of an ultrasound probe at a predetermined cycle to form measuring information including the probe angle; calculating a Doppler angle correction value corresponding to the inclination angle based on the measuring information; and calculating a corrected Doppler angle based on the Doppler angle correction value.

BRIEF DESCRIPTION OF THE DRAWINGS

FIG. 1 is a block diagram showing an illustrative embodiment of an ultrasound system.
FIG. 2 is a block diagram showing an illustrative embodiment of an ultrasound data acquisition unit.
FIG. 3 is a flow chart showing a process of correcting a Doppler angle to form a Doppler mode image.

DETAILED DESCRIPTION

This detailed description is provided with reference to the accompanying drawings. One of ordinary skill in the art may realize that the following description is illustrative only and is not in any way limiting. Other embodiments of the present invention may readily suggest themselves to such skilled persons having the benefit of this disclosure.

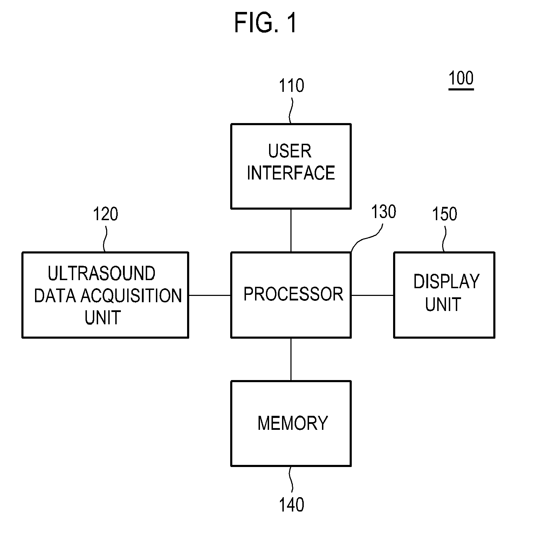

FIG. 1 is a block diagram showing an illustrative embodiment of an ultrasound system 100. The ultrasound system 100 includes a user interface 110, an ultrasound data acquisition unit 120, a processor 130, a memory 140 and a display unit 150.

The user interface 110 is configured to receive input information from a user. In one embodiment, the input information includes information for setting a region of interest on the B-mode image. The region of interest includes a sample volume for acquiring Doppler spectrum images or a color box for acquiring color Doppler images. However, it should be noted herein that the region of interest is not limited thereto.

The ultrasound data acquisition unit 120 is configured to transmit ultrasound signals to a living body. The living body includes target objects (e.g., blood flows, hearts, vascular, etc.). The ultrasound data acquisition unit 120 is further configured to receive ultrasound signals (i.e., ultrasound echo signals) reflected from the living body to acquire ultrasound data.

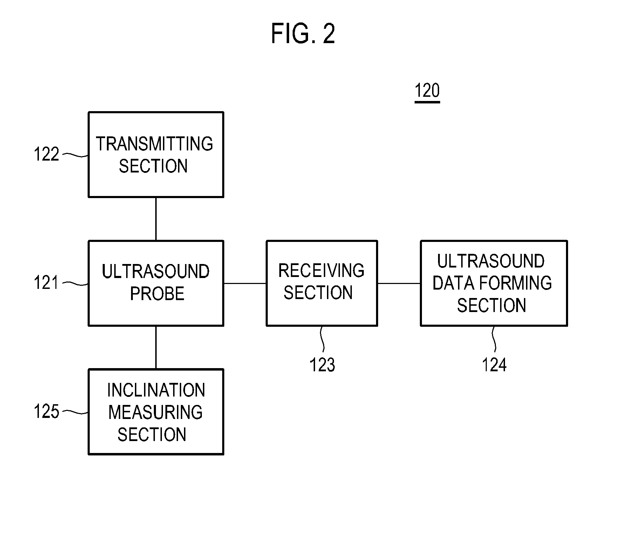

FIG. 2 is a block diagram showing an illustrative embodiment of an ultrasound data acquisition unit 120. Referring to FIG. 2, the ultrasound data acquisition unit 120 includes an ultrasound probe 121, a transmitting section 122, a receiving section 123, an ultrasound data forming section 124 and an inclination measuring section 125.

The ultrasound probe 121 includes a plurality of transducer elements (not shown) for reciprocally converting between electrical signals and the ultrasound signals. The ultrasound probe 121 is configured to transmit the ultrasound signals to the living body and receive the ultrasound echo signals reflected from the living body to output electrical signals (hereinafter, referred to as "reception signals"). The reception signals are analog signals.

The transmitting section 122 is configured to control the transmission of the ultrasound signals. The transmitting section 122 is configured to generate electrical signals (hereinafter, referred to as "transmission signals") for acquiring an ultrasound image in consideration of transducer elements and focal points.

In one embodiment, the transmitting section 122 is configured to generate first transmission signals for acquiring a B-mode image in consideration of the transducer elements and the focal points. Thus, the ultrasound probe 121 is configured to convert the first transmission signals provided from the transmitting section 122 into the ultrasound signals, transmit the ultrasound signals to the living body, and receive the ultrasound echo signals reflected from the living body to output first reception signals.

The transmitting section 122 is further configured to generate second transmission signals for acquiring a Doppler mode image corresponding to the region of interest in consideration the transducer elements and the focal points. Thus, the ultrasound probe 121 is configured to convert the second transmission signals provided from the transmitting section 122 into the ultrasound signals, transmit the ultrasound signals to the living body, and receive the ultrasound echo signals reflected from the living body to output second reception signals.

The receiving section 123 is configured to perform an analog-digital conversion upon the reception signals provided from the ultrasound probe 121 to form digital signals. The receiving section 123 is further configured to perform a reception beam-forming upon the digital signals in consideration of the transducer elements and the focal points to form reception-focused signals.

In one embodiment, the receiving section 123 is configured to perform the analog-digital conversion upon the first reception signals provided from the ultrasound probe 121 to form first digital signals. The receiving section 123 is further configured to perform the reception beam-forming upon the first digital signals in consideration of the transducer elements and the focal points to form first reception-focused signals. The receiving section 123 is also configured to perform the analog-digital conversion upon the second reception signals provided from the ultrasound probe 121 to form second digital signals. The receiving section 123 is additionally configured to perform the reception beam-forming upon the second digital signals in consideration of the transducer elements and the focal points to form second reception-focused signals.

The ultrasound data forming section 124 is configured to form ultrasound data based on the reception-focused signals provided from the receiving section 123. The ultrasound data forming section 124 is further configured to perform a signal process (e.g., gain control, etc) upon the reception-focused signals.

In one embodiment, the ultrasound data forming section 124 is configured to form first ultrasound data corresponding to the B-mode image based on the first reception-focused signals provided from the receiving section 123. The first ultrasound data includes radio frequency data. However, it should be noted herein that the first ultrasound data are not limited thereto. The ultrasound data forming section 124 is further configured to form second ultrasound data corresponding to the region of interest (i.e., Doppler mode image) based on the second reception-focused signals provided from the receiving section 123.

The inclination measuring section 125 is configured to measure an inclination angle of the ultrasound probe 121 (hereafter, referred to as a "probe angle") at a predetermined cycle to thereby form measuring information including the probe angle. The predetermined cycle represents a cycle for performing Doppler calculation, i.e., calculation of the blood flow velocities. However, it should be noted herein that the predetermined cycle is not limited thereto. The inclination measuring section 125 is mounted inside or outside of the ultrasound probe 121. However, it should be noted herein that the inclination measuring section 125 is not limited thereto. The inclination measuring section 125 is connected to the processor 130 in a wired or wireless manner. Any apparatus, which can measure the inclination angle of the ultrasound probe 121, is adopted as the inclination measuring section 125. For example, the inclination measuring section 125 includes a gyroscope, an accelerometer and the like.

In one embodiment, the inclination measuring section 125 is configured to start the measurement of the inclination angle of the ultrasound probe 121 according to the Doppler angle correction start under the control of the processor 130 to form first measuring information. Then, the inclination measuring section 125 is configured to measure the inclination angle of the ultrasound probe 121 at the predetermined cycle to form second measuring information, third measuring information, ... $n^{th}$ measuring information. Furthermore, the inclination measuring section 125 is configured to end the measurement of the inclination angle of the ultrasound probe 121 according to the Doppler angle correction end under the control of the processor 130.

In another embodiment, the inclination measuring section 125 is configured to measure the inclination angle of the ultrasound probe 121 when the ultrasound probe 121 is operated (i.e., ultrasound probe 121 is activated) to form the measuring information. Furthermore, the inclination measuring section 125 is configured to end the measurement of the inclination angle of the ultrasound probe 121 when the ultrasound probe 121 is not operated (i.e., ultrasound probe 121 is deactivated).

Referring back to FIG. 1, the processor 130 is configured to form the ultrasound image based on the ultrasound data provided from the ultrasound data acquisition unit 120.

The processor 130 includes a central processing unit, a microprocessor, a graphic processing unit and the like.

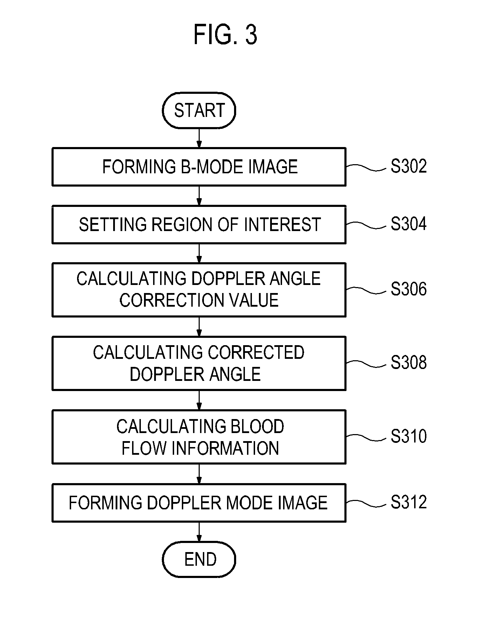

FIG. 3 is a flow chart showing a process of correcting a Doppler angle to form a Doppler mode image. The processor 130 is configured to form the B-mode image based on the first ultrasound data provided from the ultrasound data acquisition unit 120 at step S302 in FIG. 3. The B-mode image is displayed on the display unit 150. Thus, the user sets the region of interest on the B-mode image by using the user interface 110.

The processor is configured to set the region of interest on the B-mode image based on the input information provided from the user interface 110 at step S304. Thus, the ultrasound data acquisition unit 120 is configured to transmit the ultrasound signals to the living body and receive the ultrasound echo signals reflected from the living body to acquire the second ultrasound data corresponding to the region of interest.

The processor 130 is configured to calculate a Doppler angle correction value based on the measurement information provided form the inclination measuring section 125 at step S306 in FIG. 3.

In one embodiment, the processor 130 calculates the Doppler angle correction value by using the following equation:

$$\Delta\phi_n = \phi_n - \phi_0 \qquad\qquad (4)$$

wherein $\phi_n$ represents the probe angle (hereinafter, referred to as "$n^{th}$ probe angle") of the ultrasound probe 121 at a present cycle (i.e., $n^{th}$ cycle), $\phi_0$ represents an initial probe angle of the ultrasound probe 121, and $\Delta\phi_n$ represents the Doppler angle correction value at the present cycle (i.e., angle variation between initial probe angle $\phi_0$ and $n^{th}$ probe angle $\phi_n$). The initial probe angle represents the inclination angle, which is initially measured by the inclination measuring section 125, when the second ultrasound data are acquired. However, it should be noted herein that the initial probe angle is not limited thereto.

In another embodiment, the processor 130 is configured to calculate the Doppler angle correction value as equation 5 provided below.

$$\Delta\phi_n = \phi_n - \Delta\phi_{n-1} \qquad\qquad (5)$$

In equation 5, $\phi_n$ represents the $n^{th}$ probe angle of the ultrasound probe 121, $\Delta\phi_{n-1}$ represents the Doppler angle correction value (hereinafter, referred to as "$(n-1)^{th}$ Doppler angle correction value") at a previous cycle (i.e., $(n-1)^{th}$ cycle), and $\Delta\phi_n$ represents the Doppler angle correction value at the present cycle (i.e., angle variation between $n^{th}$ probe angle $\phi_n$ and $(n-1)^{th}$ Doppler angle correction value $\Delta\phi_{n-1}$).

The processor 130 is configured to calculate a corrected Doppler angle based on the Doppler angle correction value at step S308 in FIG. 3.

In one embodiment, the processor 130 is configured to calculate the corrected Doppler angle as equation 6 provided below.

$$\theta'_n = \theta_0 + \Delta\phi_n \qquad\qquad (6)$$

In equation 6, $\theta'_n$ represents the corrected Doppler angle at the present cycle, and $\theta_0$ represents an initial Doppler angle. The initial Doppler angle $\theta_0$ represents a Doppler angle that is initially set by the user or the ultrasound system.

In another embodiment, the processor 130 is configured to calculate the corrected Doppler angle as equation 7 provided below.

$$\theta'_n = \theta'_{n-1} + \Delta\phi_n \qquad\qquad (7)$$

In equation 7, $\theta'_{n-1}$ represents the corrected Doppler angle at the previous cycle.

The processor 130 is configured to calculate blood flow information (e.g., velocity of blood flow) at step S310 in FIG. 3. That is, the processor 130 is configured to calculate the velocity of the blood flow $v$ by applying the corrected

Doppler angle $\theta_n^{'}$ to equation 3.

$$v = \frac{cf_D}{2f_0 \cos\theta_n^{'}} \qquad (8)$$

The processor 130 is configured to form the Doppler mode image based on the blood flow information (e.g., velocity of blood flow) at step S312 in FIG. 4. The methods of forming the Doppler mode image are well known in the art. Thus, they have not been described in detail so as not to unnecessarily obscure the present disclosure.

Optionally, the processor 130 is configured to control the start and end of measuring the probe angle according to the Doppler angle correction start and the Doppler angle correction end by the user. Furthermore, the processor 130 is configured to control the start and end of measuring the probe angle according to the start and end of acquiring of the second ultrasound data.

Referring back to FIG. 1, the memory 140 stores the ultrasound data acquired by the ultrasound data acquisition unit 120. The memory 140 further stores the Doppler angle calculated by the processor 130. The memory 140 also stores the Doppler angle correction value calculated by the processor 130.

The display unit 150 is configured to display the B-mode image formed by the processor 130. The display unit 150 is further configured to display the Doppler mode image formed by the processor 130. The display unit 150 includes a cathode ray tube (CRT) display, a liquid crystal display (LCD), an organic light emit diode (OLED) display and the like.

Any reference in this specification to "one embodiment," "an embodiment," "example embodiment," "illustrative embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to affect such feature, structure or characteristic in connection with other embodiments.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

**Claims**

1.  An ultrasound system, comprising:

    an inclination measuring unit configured to measure an inclination angle of an ultrasound probe at a predetermined cycle to form measuring information including the inclination angle, wherein the inclination measuring unit is mounted inside or outside of the ultrasound probe; and
    a processing unit configured to calculate a Doppler angle correction value corresponding to the inclination angle based on the measuring information and calculate a corrected Doppler angle based on the Doppler angle correction value.

2.  The ultrasound system of Claim 1, wherein the processor is configured to calculate an angle variation between an initial inclination angle of the ultrasound probe and an inclination angle of the ultrasound probe at a preset cycle as the Doppler angle correction value based on the measuring information.

3.  The ultrasound system of Claim 2, wherein the processor is configured to calculate the corrected Doppler angle based on the Doppler angle correction value at the present cycle and an initial Doppler angle.

4.  The ultrasound system of Claim 1, wherein the processor is configured to calculate an angle variation between an inclination angle of the ultrasound probe at a present cycle and the an inclination angle of the ultrasound probe at a previous cycle as the Doppler angle correction value based on the measuring information.

5. The ultrasound system of Claim 4, wherein the processor is configured to calculate the corrected Doppler angle based on the Doppler angle correction value at the present cycle and the corrected Doppler angle at the previous cycle.

6. A method of correcting a Doppler angle, comprising:

   a) measuring an inclination angle of an ultrasound probe at a predetermined cycle to form measuring information including the inclination angle;
   b) calculating a Doppler angle correction value corresponding to the inclination angle based on the measuring information; and
   c) calculating a corrected Doppler angle based on the Doppler angle correction value.

7. The method of Claim 6, wherein step b) comprises:

   calculating an angle variation between an inclination angle of the ultrasound probe at a present cycle and an inclination angle of the ultrasound probe at a previous cycle as the Doppler angle correction value based on the measuring information.

8. The method of Claim 7, wherein step c) comprises:

   calculating the corrected Doppler angle based on the Doppler angle correction value at the present cycle and an initial Doppler angle.

9. The method of Claim 6, wherein step b) comprises:

   calculating an angle variation between an inclination angle of the ultrasound probe at a present cycle and an inclination angle of the ultrasound probe at a previous cycle as the Doppler angle correction value based on the measuring information.

10. The method of Claim 9, wherein step c) comprises:

   calculating the corrected Doppler angle based on the Doppler angle correction value at the present cycle and the corrected Doppler angle at the previous cycle.

# FIG. 1

100

110

USER
INTERFACE

120

ULTRASOUND
DATA ACQUISITION
UNIT

130

PROCESSOR

150

DISPLAY
UNIT

MEMORY

140

# FIG. 2

120

```
         ┌─────────────────┐
122 ─────│  TRANSMITTING   │
         │     SECTION     │
         └─────────────────┘
                  │
         ┌─────────────────┐      ┌──────────────┐      ┌──────────────────┐
121 ─────│   ULTRASOUND    │──────│  RECEIVING   │──────│    ULTRASOUND    │
         │     PROBE       │      │   SECTION    │      │  DATA FORMING    │
         └─────────────────┘      └──────────────┘      │     SECTION      │
                  │                      │              └──────────────────┘
         ┌─────────────────┐            123                      │
         │   INCLINATION   │                                    124
125 ─────│    MEASURING    │
         │     SECTION     │
         └─────────────────┘
```

# FIG. 3

```
                    ┌─────────┐
                    │  START  │
                    └─────────┘
                         │
                         ▼
    ┌──────────────────────────────────────┐
    │       FORMING B-MODE IMAGE           │────  S302
    └──────────────────────────────────────┘
                         │
                         ▼
    ┌──────────────────────────────────────┐
    │      SETTING REGION OF INTEREST      │────  S304
    └──────────────────────────────────────┘
                         │
                         ▼
    ┌──────────────────────────────────────┐
    │     CALCULATING DOPPLER ANGLE        │────  S306
    │         CORRECTION VALUE             │
    └──────────────────────────────────────┘
                         │
                         ▼
    ┌──────────────────────────────────────┐
    │       CALCULATING CORRECTED          │────  S308
    │          DOPPLER ANGLE               │
    └──────────────────────────────────────┘
                         │
                         ▼
    ┌──────────────────────────────────────┐
    │        CALCULATING BLOOD             │────  S310
    │        FLOW INFORMATION              │
    └──────────────────────────────────────┘
                         │
                         ▼
    ┌──────────────────────────────────────┐
    │     FORMING DOPPLER MODE IMAGE       │────  S312
    └──────────────────────────────────────┘
                         │
                         ▼
                    ┌─────────┐
                    │   END   │
                    └─────────┘
```

Europäisches Patentamt

European Patent Office

Office européen des brevets

**EUROPEAN SEARCH REPORT**

Application Number

EP 12 17 7809

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 701 898 A (ADAM DAN R [IL] ET AL) 30 December 1997 (1997-12-30) * abstract * * figures 1-3 * * column 2, line 66 - column 9, line 53 * | 1-10 | INV. A61B8/06 A61B8/08 |
| X | US 4 127 842 A (HASSLER DIETER) 28 November 1978 (1978-11-28) * abstract * * figures 1,2 * * column 3, line 31 - column 8, line 12 * | 1,6 | |
| A | US 5 109 858 A (NISHIYAMA HISASHI [JP] ET AL) 5 May 1992 (1992-05-05) * abstract * * figure 4 * * column 8, line 29 - column 8, line 50 * | 3-5,8-10 | |

TECHNICAL FIELDS SEARCHED (IPC)

A61B

The present search report has been drawn up for all claims

| Place of search | Date of completion of the search | Examiner |
|---|---|---|
| The Hague | 24 October 2012 | Moehrs, Sascha |

**ANNEX TO THE EUROPEAN SEARCH REPORT
ON EUROPEAN PATENT APPLICATION NO.**                    EP 12 17 7809

This annex lists the patent family members relating to the patent documents cited in the above-mentioned European search report.
The members are as contained in the European Patent Office EDP file on
The European Patent Office is in no way liable for these particulars which are merely given for the purpose of information.

24-10-2012

| Patent document cited in search report | | Publication date | Patent family member(s) | | Publication date |
|---|---|---|---|---|---|
| US 5701898 | A | 30-12-1997 | NONE | | |
| US 4127842 | A | 28-11-1978 | AT | 369547 B | 10-01-1983 |
| | | | DE | 2555134 B1 | 05-05-1977 |
| | | | FR | 2334957 A1 | 08-07-1977 |
| | | | GB | 1551345 A | 30-08-1979 |
| | | | JP | 1179938 C | 30-11-1983 |
| | | | JP | 52071277 A | 14-06-1977 |
| | | | JP | 58007289 B | 09-02-1983 |
| | | | NL | 7609350 A | 10-06-1977 |
| | | | US | 4127842 A | 28-11-1978 |
| US 5109858 | A | 05-05-1992 | JP | 3228752 A | 09-10-1991 |
| | | | US | 5109858 A | 05-05-1992 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020110074257